# EUROPEAN PATENT APPLICATION

(11) **EP 2 682 365 A1**
(43) Date of publication of application: **08.01.2014**
(21) Application number: 12175397.4
(22) Date of filing: 06.07.2012
(51) Int. Cl.: C01B 21/14, C05B 7/00, C05D 9/02, C07C 249/08

(54) **Process for the production of hydroxylamine by reduction of nitrate or nitrogen monoxide**

(71) Applicant: DSM IP Assets B.V., 6411 TE Heerlen (NL)
(72) Inventor: Tinge, Johan Thomas, 6100 AA Echt (NL); Olzheim, Daniël Julius Maria, 6100 AA Echt (NL)
(74) Representative: Duffy, James Edwin Marsh

(57) **Abstract**

The present invention provides a process for the continuous production of hydroxylamine, comprising
i) feeding to a hydroxylamine formation zone:
a) hydrogen-containing gas;
b) phosphate-containing acidic aqueous solution; and
c) nitrate or nitrogen monoxide dissolved in said

phosphate-containing acidic aqueous solution;
ii) reducing, in said hydroxylamine formation zone, nitrate or nitrogen monoxide with hydrogen to form hydroxylamine;
iii) removing hydroxylamine from said phosphate-containing acidic aqueous solution, in a hydroxylamine removal zone;
iv) adding to said phosphate-containing acidic aqueous solution nitrous gases and/or nitrate; and
v) returning to said hydroxylamine formation zone said phosphate-containing acidic aqueous solution;

characterized in that a portion of said phosphate-containing acidic aqueous solution is removed; and phosphoric acid is added to the remaining phosphate-containing acidic aqueous solution;
a process for preparing oximes therefrom; a process for preparing lactams therefrom; and a fertilizer product produced therefrom.

## Description

The invention relates to a process for the production of hydroxylamine by the reduction of nitrate or nitrogen monoxide; to a process for preparing oximes therefrom; to a process for preparing lactams therefrom; and to a fertilizer product produced therefrom.

Hydroxylamine is a commonly used reagent in a myriad of organic and inorganic reactions. It is in particular suitable for use in the preparation of oximes, in particular cyclohexanone oxime, which may thereafter be converted into caprolactam via a Beckmann rearrangement. Beckmann rearrangement processes for the preparation of caprolactam are generally known in the art, e.g. from Ullmann's encyclopedia of Industrial Chemistry, for instance the 7th edition (2005), Chapter on Caprolactam (DOI: 10.1002/14356007.a05.031 (Retrieved Feb. 18, 2011). Other oximes of which the preparation using hydroxylamine has been described include cyclododecanone oxime (e.g. EP-A 1 329 448) and butanone oxime.

Methods of preparing hydroxylamine are also commonly known in the art. Further, various patent rights have been published on this subject. GB-A-1,287,303 and US-5,364,609, for example, relate to processes wherein nitrate is reduced in a phosphate buffer solution using molecular hydrogen.

The HPO^{®} cyclohexanone oxime process of DSM (see *e.g*. H.J. Damme, J.T. van Goolen and A.H. de Rooij, Cyclohexanone oxime made without by-product (NH4)2SO4, July 10, 1972, Chemical Engineering; pp 54/55 or Ullmann's encyclopedia of industrial chemistry (2005), under the Chapter Caprolactam, p 6/7; DOI: 10.1002/14356007.a05.031 (Retrieved Feb. 18, 2011) makes use of two recycling liquids - an inorganic liquid and an organic liquid - in which several reactions and operations take place. The inorganic liquid, a phosphate-containing acidic aqueous solution, also containing ammonium, nitrate and/or nitrogen monoxide, is fed to a hydroxylamine formation zone, where hydroxylamine is produced. Hydroxylamine is formed via reduction of nitrate with hydrogen, which is catalysed by a heterogeneous catalyst, for example a palladium-containing catalyst with carbon as carrier.

In the case where the hydroxylamine formation starts from a solution of phosphoric acid and nitrate the chemical reactions occurring are represented as follows:
Reaction 1) Preparation of the hydroxylamine in the hydroxylamine formation zone:

   2 H₃PO₄ + NO₃⁻ + 3 H₂ → NH₃OH⁺ + 2 H₂PO₄⁻ + 2 H₂O

This reaction is catalyzed heterogeneously. Typically, the catalyst is present as a disperse phase of finely divided solids in a liquid reaction mixture. The resulting mixture of the first reaction is a phosphate-containing acidic aqueous solution comprising a suspension of solid catalyst particles in a hydroxylamine solution.

In the HPO^{®} cyclohexanone oxime process of DSM, hydroxylamine is further reacted to form cyclohexanone oxime by the following reaction:
Reaction 2) Preparation of the cyclohexanone oxime in the hydroxylamine removal zone (cyclohexanone oxime section):

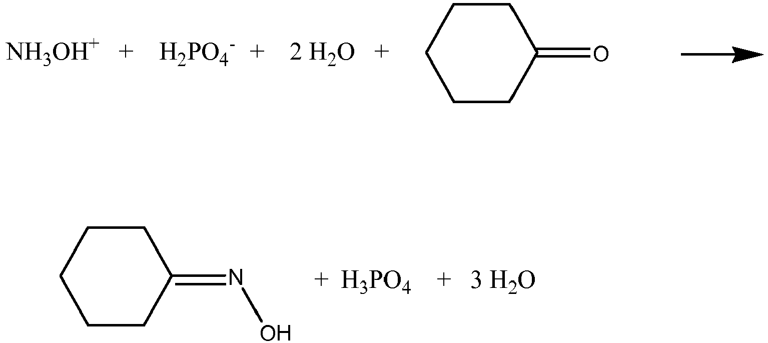

After separation of the cyclohexanone oxime from the solution of phosphoric acid, fresh nitrate or nitrogen oxides are added, to the solution of phosphoric acid, before it is recycled to the hydroxylamine formation zone.
Reaction 3) Supply of HNO₃ to make up the depletion of the source of nitrate ions:

   H₃PO₄ + H₂PO₄⁻ + HNO₃ + 3 H₂O → 2 H₃PO₄ + NO₃⁻ + 3 H₂O

Although the preparation of hydroxylamine has been known for many decades and ways to improve known preparation methods have been investigated thoroughly over the years, presently known industrial processes still suffer from drawbacks.

In particular, in known continuous processes the selectivity towards the conversion into the by-products ammonium, N₂O and N₂ is a problem. The selectivity towards these by-products increases with time.

The by-products may be formed according to the following equations:

NO₃⁻ + 2 H⁺ + 4 H₂ → NH₄⁺ + 3 H₂O

2 NO₃⁻ + 2 H⁺ + 4 H₂ → N₂O + 5 H₂O

2 NO₃⁻ + 2 H⁺ + 5 H₂ → N₂ + 6 H₂O

A problem with the above process is that the catalyst for reduction to hydroxylamine is particularly sensitive to poisoning, especially by metals such as molybdenum which are typically present in steel used for the manufacture of reactor components. A.H. de Rooij et al describe in Chemtech, Volume 7, May 1977, pp 309-315 that this problem was solved by using type 304 rather than type 316 stainless steel. However, the conditions of phosphoric acid and nitric acid in the phosphate-containing acidic aqueous solution cause corrosion, even in low-molybdenum steel equipment. Therefore, molybdenum is dissolved into the phosphate-containing acidic aqueous solution. In addition flows charged to the hydroxylamine formation zone, for example nitric acid and phosphoric acid might contain molybdenum. It has been estimated that the presence of 1 ppm molybdenum in the phosphate-containing acidic aqueous solution can lead to a reduction in hydroxylamine selectivity of as much as 2%.

One solution to this problem is to remove molybdenum from the nitrate/nitrogen monoxide solution by co-precipitation with a complex iron-ammonium phosphate. Such a process is described in US patent no. 4,062,927. After reducing the hydroxylamine concentration to less than 0.2 mol/kg, iron, ammonium and phosphate salts are added. The resulting iron-ammonium phosphate/molybdenum salt is separated. A drawback of this process is that the dilution of the hydroxylamine solution, addition of complexing agent, and separation and removal of the complexes make the process more complicated. And, in addition, rather high investments are needed for extra process units for the addition, the precipitation, the separation and the removal of the precipitates. These investments have a negative impact on the fixed costs for the production of hydroxylamine and the products produced therefrom.

Japanese patent no. 52-051340 describes reducing molybdenum concentration by treating the inorganic liquid with an ion exchange resin having a chelate forming group, for example thioamide chelate resin, thiol chelate resin, dithoic acid chelate resin and carboxylic acid chelate resin. The aqueous solution is passed through either a horizontal or vertical ion exchange resin packed bed either batchwise or continuously. A drawback of this process is that an additional process unit is required for loading and unloading of the ion exchange material. As a result of implementing this technology, the required investments for the production of hydroxylamine will increase, having a negative impact on the fixed costs for the production of hydroxylamine and the products produced therefrom. In addition, the ion exchange resin will also be consumed, and its regeneration with alkali complicates the overall process. Due to the costs of both ion exchange resin and alkali the variable costs for the production of hydroxylamine and the products produced thereof will increase.

In view of the above, it is an object of the present invention to provide a method for preparing hydroxylamine with a high degree of selectivity, in a more efficient and more economical manner.

The present inventors have found an alternative solution to the problem of molybdenum build-up which has the further advantage that the added cost and complexity of the above methods is avoided. Specifically they have developed a process which involves reducing the molybdenum concentration in the inorganic liquid by regularly draining a portion of the phosphate-containing acidic aqueous solution containing molybdenum and replacing it with fresh phosphoric acid. Accordingly, the present invention provides a process for the continuous production of hydroxylamine, comprising
i) feeding to a hydroxylamine formation zone:
   a) hydrogen-containing gas;
   b) phosphate-containing acidic aqueous solution; and
   c) nitrate or nitrogen monoxide dissolved in said phosphate-containing acidic aqueous solution;
ii) reducing, in said hydroxylamine formation zone, nitrate or nitrogen monoxide with hydrogen to form hydroxylamine;
iii) removing hydroxylamine from said phosphate-containing acidic aqueous solution, in a hydroxylamine removal zone;
iv) adding to said phosphate-containing acidic aqueous solution nitrous gases and/or nitrate; and
v) returning to said hydroxylamine formation zone said phosphate-containing acidic aqueous solution;
**characterized in that** a portion of said phosphate-containing acidic aqueous solution is removed; and phosphoric acid is added to the remaining phosphate-containing acidic aqueous solution.

The above process reduces the concentration of molybdenum in the phosphate-containing acidic aqueous solution. As mentioned above this improves the selectivity of the reaction towards the formation of hydroxylamine. Accordingly, the raw material consumption (nitrate and/or nitrous gases and hydrogen) of the process is reduced, as is the energy consumption. Both have beneficial effects on the environment and the carbon foot print of the process. An additional advantage of the above process is the removal of other unwanted components from the acidic aqueous solution of phosphate ions. This further improves the efficiency of the process, and reduces the energy consumption.

The phosphate-containing acidic aqueous solution includes species dissolved therein, for example nitrate. Reaction by-products, for example ammonia, may also become dissolved in the phosphate-containing acidic aqueous solution. Trace amounts of molybdenum, typically at ppm level, are also present in the phosphate-containing acidic aqueous solution.

As is clear from the reaction processes the phosphate-containing acidic aqueous solution may comprise neutral species e.g. hydroxylamine or ammonia which may also be protonated e.g. hydroxylammonium or ammonium. Accordingly, reference herein to hydroxylamine means hydroxylamine and/or hydroxylammonium ion and/or salts. Similarly, reference to ammonia means ammonia and/or ammonium ion and/or salts. Reference to phosphate means phosphoric acid, phosphate ions and/or salts, including fully and partially protonated phosphoric acid salts.

Nitrous gases and/or nitrate are added to the phosphate-containing acidic aqueous solution. In the case that nitrous gases are added, they are dissolved in the phosphate-containing acidic aqueous solution, and may form nitrate (a starting material of the hydroxylamine formation reaction). The nitrous gases required are typically produced in an ammonia combustion unit.

Phosphate-containing acidic aqueous solution, also containing dissolved nitrate, and/or nitrogen monoxide is fed to the hydroxylamine formation zone. Accordingly, the process of the present invention involves a circulating flow of phosphate-containing acidic aqueous solution. This flows from the hydroxylamine formation zone; to the hydroxylamine removal zone; to the points wherein nitrous gases and/or nitrate are added; and back to the hydroxylamine formation zone.

A portion of phosphate-containing acidic aqueous solution is removed either periodically or continuously. In this way molybdenum, and any other species dissolved in the phosphate-containing acidic aqueous solution are removed from the system. Typically the system has enough hold-up of phosphate-containing acidic aqueous solution, that it can operate with a reduced amount the of phosphate-containing acidic aqueous solution. Preferably said portion of phosphate-containing acidic aqueous solution is removed periodically.

The phosphate-containing acidic aqueous solution is removed together with whatever species it contains. Therefore, it is preferred to remove it after the hydroxylamine is already removed for further use, but before nitrous gases and/or nitrate is added to the remaining phosphate-containing acidic aqueous solution. Accordingly, the process is one wherein said portion of phosphate-containing acidic aqueous solution is removed between steps iii) and iv).

Phosphoric acid is added to the system, either periodically or continuously. It is not necessary that the amount of added phosphoric acid is equivalent to the amount of phosphate-containing acidic aqueous solution removed, because of the hold-up of phosphate-containing acidic aqueous solution in the system. However, over its lifetime, enough phosphoric acid must be added to replace the phosphate-containing acidic aqueous solution removed. The portion of phosphate-containing acidic aqueous solution is therefore effectively replaced by fresh phosphoric acid. Phosphoric acid is preferably added after phosphate-containing acidic aqueous solution is removed, to increase effectiveness of the process. It can be seen from the above reactions that water is generated both by the formation of hydroxylamine and by-products and the formation of oxime. Accordingly, the system generates water such that the added phosphoric acid becomes a phosphate-containing acidic aqueous solution.

Typically said phosphoric acid contains less than 3 ppm (weight/weight) molybdenum. Preferably, said phosphoric acid contains less than 2 ppm (weight/weight) molybdenum; more preferably less than 1 (weight/weight) molybdenum. Molybdenum concentration of the phosphoric acid may be determined by any suitable means, for example inductively coupled plasma atomic emission spectroscopy (ICP-AES).

Where phosphate-containing acidic aqueous solution is removed periodically, and phosphoric acid is added periodically, a removal-addition cycle is formed. This means a portion of phosphate-containing acidic aqueous solution is removed and subsequently an amount of phosphoric acid is added. The addition may however overlap in time with the removal. The quantity of phosphoric acid added in a single cycle need not be equivalent to the quantity of phosphate-containing acidic aqueous solution removed. The removal-addition cycle typically occurs over a period of from 1 to 10 days; preferably 2 to 8 days, for example 3, 4 or 5 days. Typically it occurs every 1 to 4 months, for example every 1, 2 or 3 months, of operation of a plant. However, frequency and duration are determined, amongst other factors, by molybdenum concentration of the circulating phosphate-containing acidic aqueous solution.

Typically, in a single removal-addition cycle, the portion of said phosphate-containing acidic aqueous solution removed is up to 25% by weight of the total phosphate-containing acidic aqueous solution.

A measurement system may be used to monitor the concentration of molybdenum in the phosphate-containing acidic aqueous solution. A control and feedback system may be used to indicate or even automatically operate the process of removing a portion of the phosphate-containing acidic aqueous solution. Molybdenum concentration of the phosphate-containing acidic aqueous solution may be determined by any suitable means, for example inductively coupled plasma atomic emission spectroscopy (ICP-AES). Typically said portion of phosphate-containing acidic aqueous solution is removed when the concentration of molybdenum in the phosphate-containing acidic aqueous solution is higher than 0.5 ppm (weight/weight). Preferably, it is removed when the concentration of molybdenum in the phosphate-containing acidic aqueous solution is higher than 1.0 ppm (weight/weight); more preferably 1.5 ppm (weight/weight).

As used herein, 'hydroxylamine selectivity' (the selectivity towards the production of hydroxylamine) is defined as follows: molar ratio of twice the amount of hydroxylamine produced in the hydroxylamine formation zone divided by the amount of H⁺ consumed in the hydroxylamine formation zone.

Fresh hydrogen is fed to the gas present in the hydroxylamine formation zone to form the hydrogen containing gas. A small amount of hydrogen containing gas is purged from the system to maintain a constant hydrogen pressure. Inert gaseous components present in the fresh hydrogen and the produced gaseous by-products N₂ and N₂O are removed via a gas purge.

Ammonia is generated as a by-product in the hydroxylamine formation. Build-up of ammonia in the phosphate-containing acidic aqueous solution should be prevented. Typically a fraction of the ammonia present in the phosphate-containing acidic aqueous solution is oxidized, in an ammonia conversion zone, by reaction with nitrous gases. N₂ gas results, which can be discharged via the gas purge. The ammonia conversion zone is typically located after the point at which a portion of phosphate-containing acidic aqueous solution is removed. Accordingly, dissolved ammonia is also removed, which saves processing it further. Preferably, said ammonia conversion zone is located after the hydroxylamine removal zone and the point where said phosphate-containing acidic aqueous solution is removed. In this way, some ammonia is conveniently removed avoiding the need for processing it further. A fraction of the remaining ammonia is treated with nitrous gases as described above, and then passes to the hydroxylamine formation zone.

Water is generated as a by-product in the hydroxylamine formation and in the oxime formation. Build-up of water in the phosphate-containing acidic aqueous solution should be prevented. Typically water present in the phosphate-containing acidic aqueous solution is removed in a water removal section, e.g. by stripping, which consumes energy. Due to the removal of a portion of phosphate-containing acidic aqueous solution less water has to be removed in the water removal section. This will reduce the overall energy consumption of the produced oxime.

The portion of phosphate-containing acidic aqueous solution removed is not required for the further reaction. It is highly acidic. Therefore, depending to what further use it is put, it might require neutralization. Typically said removed portion of said phosphate-containing acidic aqueous solution is neutralized by reaction with ammonia. The resulting solution is rich in phosphate and ammonia. It also contains ppm amounts or lower of molybdenum. Molybdenum is one of 6 "minor" elements essential for plant growth. This makes the resulting neutralized solution a very good source of fertilizer, especially for plants which are deficient in molybdenum, for example those grown in areas of acidic soil. Preferably, said neutralized phosphate-containing acidic aqueous solution is prepared as a fertilizer product.

The present invention further provides a fertilizer product comprising a phosphate-containing acidic aqueous solution obtainable by a process described herein and which has been further neutralized with ammonia. Said fertilizer product may be characterized by its molybdenum concentration. Typically, it has a molybdenum concentration of at least 1 ppm, based on the weight of the phosphate-containing acidic aqueous solution. Typically, it has a molybdenum concentration of at least 2 ppm, based on the weight of the phosphate-containing acidic aqueous solution.

The phosphate-containing acidic aqueous solution leaving the hydroxylamine formation zone should be filtered to remove catalyst. The solution obtained after filtration is then passed to the hydroxylamine removal zone.

Hydroxylamine may be removed from phosphate-containing acidic aqueous solution by selective crystallization or chemical conversion. Typically hydroxylamine is removed in the hydroxylamine removal zone by reaction with a ketone to form an oxime. In this case the hydroxylamine removal zone may also be known as an oximation section. Typically, a ketone is provided in an organic phase, for example toluene or benzene. Alternatively, cyclohexanone is added as such and is mixed in the hydroxylamine removal zone with for example toluene or benzene. The resulting oxime typically is discharged from the hydroxylamine removal zone in the organic phase.

Preferably, said ketone is cyclohexanone, cyclododecanone or butanone, and said oxime is, respectively, cyclohexanone oxime, cyclododecanone oxime or butanone oxime. More preferably, the ketone is cyclohexanone, and the oxime is cyclohexanone oxime. Typically, the supplied organic phase is a mixture of toluene and cyclohexanone. Here the cyclohexanone is practically quantitatively converted into cyclohexanone oxime. The obtained cyclohexanone oxime-containing organic phase is distilled to recover the toluene.

The phosphate-containing aqueous acidic solution leaving the hydroxylamine removal zone has to be purified thoroughly to protect the catalyst in the hydroxylamine formation zone. This is done by extraction with toluene or benzene, followed by stripping with steam. In the stripping column water that is co-produced during the preparation of hydroxylamine and cyclohexanone oxime is also removed. This typically forms a part of the oximation section itself.

Further preferred is that said ketone is cyclohexanone or cyclododecanone and the resulting cyclohexanone oxime or cyclododecanone oxime is further reacted by Beckmann rearrangement to form, respectively, caprolactam or laurolactam. More preferably the oxime is cyclohexanone oxime, and caprolactam is produced.

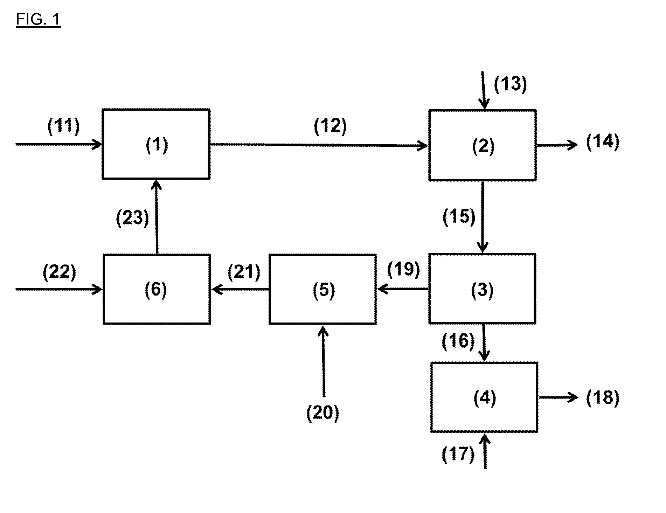

FIG. 1 describes a preferred embodiment of the present invention. Hydrogen-containing gas is provided through line (11) to hydroxylamine formation zone (1). Phosphate-containing acidic aqueous solution is provided via line (23) as is nitrate dissolved in the phosphate-containing acidic aqueous solution. The phosphate-containing acidic aqueous solution also containing hydroxylamine is passed though line (12) to hydroxylamine removal zone (2), also known as an oximation section. Ketone is provided through line (13). After reaction, the resulting oxime is removed in organic phase through line (14). The phosphate-containing acidic aqueous solution, is passed through line (15) to separating unit (3). Periodically a portion of the phosphate-containing acidic aqueous solution is removed via line (16) to neutralization section (4). Ammonia supplied via line (17) is used for neutralization. The resulting neutralized solution is removed via line (18). The remaining phosphate-containing acidic aqueous solution is passed via line (19) to ammonia conversion zone (5), where nitrous gases supplied via line (20) are used to convert ammonia, therein. The obtained solution with reduced ammonia content passes via line (21) to addition unit (6) where nitrous gases and/or nitrate are added via line (22) and dissolved in the solution. The resulting solution is fed via line (23) to hydroxylamine formation zone (1), and the process continues in a loop.

In case nitrogen oxide is used as feedstock instead of nitrate and/or nitrous gases, line (22) and addition zone (6) are not required; rather a nitrogen monoxide-containing gas will be fed directly to hydroxylamine formation zone (1).

In another embodiment, cyclohexanone is added as such to the oximation section (2), in which it will be mixed with toluene.

The present invention is illustrated by but not limited to the following example.

### EXAMPLE

This experiment was performed in a commercial cyclohexanone oxime plant, operating according to DSM's HPO^{®} process. Nitrate was continuously and selectively reduced with hydrogen gas to form hydroxylamine. The produced hydroxylamine was converted into cyclohexanone oxime in an oximation section by reaction with cyclohexanone. The set-up was essentially depicted as in FIG. 1.

The HPO^{®} plant contained a hydroxylamine formation zone in which nitrate, present in an aqueous phosphate buffered solution, was reduced. As heterogeneous catalyst Pd/C was applied and GeO₂ was added as promoter. A part of the phosphate-containing acidic aqueous solution, also containing hydroxylamine was continuously discharged and was, after removal of the catalyst by filtration, charged to the oximation section. In the oximation section the aqueous hydroxylamine solution was contacted with cyclohexanone dissolved in toluene. The oximation section was operated at a temperature of approx. 50 °C. The majority of the hydroxylamine was converted to cyclohexanone oxime. The obtained cyclohexanone oxime was, after being water-washed and distilled, charged into the Beckmann rearrangement section in which it was, in the presence of oleum, converted into caprolactam. The obtained caprolactam-containing reaction mixture was neutralized with ammonia and the raw caprolactam was further purified by extraction and distillation.

The aqueous solution remaining after oximation had a reduced content of hydroxylamine, and was extracted with toluene in order to recover both unreacted cyclohexanone and produced cyclohexanone oxime. After this extraction the remaining aqueous solution was charged to a steam stripper column, in which both toluene and part of the water were removed overhead. The bottom flow of this stripper was charged to the hydroxylamine formation zone after partial removal of by-product ammonia and charging of nitrate.

Just prior to the start of the experiment the hydroxylamine selectivity of the nitrate reduction was 81.1 % measured by titration and the molybdenum concentration of the aqueous solution after filtration of the Pd/C catalyst was 3.25 ppm (weight/weight), as measured by ICP-AES. In the hydroxylamine formation zone, the temperature was approximately 49 °C; the total gas pressure leaving the hydroxylamine formation zone was 2.2 MPa; the hydrogen fraction in the gas leaving the hydroxylamine formation zone was 60 % by volume; and the hydroxylamine concentration of the phosphate-containing acidic aqueous solution leaving the hydroxylamine formation zone was about 1.2 mol/kg.

[H⁺], [hydroxylamine], [phosphate] and [ammonia] concentrations were all determined by equilibrium titration of a sample, by subsequently titrating a sample of the solution to a pH of 4.2 at 25 °C with 0.25 N aqueous NaOH solution to get the [H⁺] concentration ("free acid") at the first equilibrium point (at a pH of about 4.2) equilibrium titration was continued so as to subsequently reach three further equivalence points, the first of which corresponds to the free acid coming from hydroxylamine (and thus provides the value for [hydroxylamine] in the sample); the second of which provides the value for [phosphate], and the last of which provides a value for [ammonia].

During the experiment approximately 68 tons of the bottom flow from the steam stripper column aqueous solution was discharged from the aqueous process loop to a neutralization section, from a total of approximately 550 tons of phosphate-containing acidic aqueous solution in the system. The removed bottom flow had a concentration of molybdenum of 3.89 ppm (weight/weight). In order to compensate for the removal of phosphate from the aqueous process loop, 47.1 tons of fresh aqueous H₃PO₄ solution was charged to the aqueous process loop, prior to feeding to the hydroxylamine formation zone. The molybdenum concentration of this fresh aqueous H₃PO₄ solution was 0.60 ppm (weight/weight), as measured by ICP-AES. The removal-addition cycle took approximately 5 days, after which time, the molybdenum concentration of the aqueous solution after filtration of the Pd/C catalyst was reduced to 2.55 ppm (weight/weight), as measured by ICP-AES. Due to generation of water, and addition of other reagents, after the removal-addition cycle, the total phosphate-containing acidic aqueous solution was approximately 615 tons.

Directly after the experiment was completed the hydroxylamine selectivity of the nitrate reduction was 82.6 %. The temperature; the total gas pressure leaving the hydroxylamine formation zone; hydrogen fraction in the gas leaving the hydroxylamine formation zone; and hydroxylamine concentration of the phosphate-containing acidic aqueous solution leaving the hydroxylamine formation zone were almost identical to those prior to the experiment.

In the neutralization section ammonia water was added in small batches to the discharged aqueous bottom stripper solution. During this step the temperature of the neutralized aqueous solution was kept below 71 °C. Ammonia water was added until the pH was approx. 7.0. The obtained neutralized aqueous solution was applied as fertilizer.

The Example demonstrates that, due to the reduction of the molybdenum concentration from 3.25 to 2.55 ppm (weight/weight) the hydroxylamine selectivity of the nitrate reduction was increased from 81.1 to 82.6 %. The Example further demonstrates that a valuable high grade liquid molybdenum containing NP-fertilizer can be produced.

## Claims

1. A process for the continuous production of hydroxylamine, comprising
i) feeding to a hydroxylamine formation zone:
a) hydrogen-containing gas;
b) phosphate-containing acidic aqueous solution; and
c) nitrate or nitrogen monoxide dissolved in said phosphate-containing acidic aqueous solution;
ii) reducing, in said hydroxylamine formation zone, nitrate or nitrogen monoxide with hydrogen to form hydroxylamine;
iii) removing hydroxylamine from said phosphate-containing acidic aqueous solution, in a hydroxylamine removal zone;
iv) adding to said phosphate-containing acidic aqueous solution nitrous gases and/or nitrate; and
v) returning to said hydroxylamine formation zone said phosphate-containing acidic aqueous solution;
**characterized in that** a portion of said phosphate-containing acidic aqueous solution is removed; and phosphoric acid is added to the remaining phosphate-containing acidic aqueous solution.

2. A process according to claim 1, wherein said portion of phosphate-containing acidic aqueous solution is removed periodically.

3. A process according to claim 2, wherein said phosphoric acid is added periodically, such that there is a removal-addition cycle.

4. A process according to claim 3, wherein, in a single removal-addition cycle, the portion of said phosphate-containing acidic aqueous solution removed is up to 25% by weight of the total phosphate-containing acidic aqueous solution.

5. A process according to any one of clams 1 to 4, wherein said portion of phosphate-containing acidic aqueous solution is removed between steps iii) and iv).

6. A process according to any one of claims 1 to 5, wherein said portion of phosphate-containing acidic aqueous solution is removed when the concentration of molybdenum in the phosphate-containing acidic aqueous solution is higher than 1 ppm (weight/weight).

7. A process according to any one of claims 1 to 6, wherein ammonia present in the phosphate-containing acidic aqueous solution is oxidized, in an ammonia conversion zone, by reaction with nitrous gases.

8. A process according to claim 7, wherein said ammonia conversion zone is located after the hydroxylamine removal zone and the point where said phosphate-containing acidic aqueous solution is removed.

9. A process according to any one of claims 1 to 8, wherein said removed portion of said phosphate-containing acidic aqueous solution is neutralized by reaction with ammonia.

10. A process according to claim 9, wherein said neutralized phosphate-containing acidic aqueous solution is prepared as a fertilizer product.

11. A process according to any one of claims 1 to 10, wherein said phosphoric acid contains less than 3 ppm (weight/weight) molybdenum.

12. A process according to any one of claims 1 to 11, wherein hydroxylamine is removed in the hydroxylamine removal zone by reaction with a ketone to form an oxime.

13. A process according to claim 12, wherein said ketone is cyclohexanone, cyclododecanone or butanone, and said oxime is, respectively, cyclohexanone oxime, cyclododecanone oxime or butanone oxime.

14. A process according to claim 12 or 13, wherein said ketone is cyclohexanone or cyclododecanone and the resulting cyclohexanone oxime or cyclododecanone oxime is further reacted by Beckmann rearrangement to form, respectively, caprolactam or laurolactam.

15. A fertilizer product comprising a phosphate-containing acidic aqueous solution, obtainable by a process according to any one of claims 1 to 14, which is neutralized by reaction with ammonia.
